# EUROPEAN PATENT APPLICATION

(11) **EP 4 389 203 A1**
(43) Date of publication of application: **26.06.2024**
(21) Application number: 22215090.6
(22) Date of filing: 20.12.2022
(51) Int. Cl.: A61N 5/06

(54) **FLEXIBLE CAP DEVICE**

(71) Applicant: Koninklijke Philips N.V., 5656 AG Eindhoven (NL)
(72) Inventor: LELIEVELD, Mark Johannes, Eindhoven (NL); LAM, Hiu Man, Eindhoven (NL); NUIJS, Antonius Maarten, 5656AG Eindhoven (NL); BROWN, Guy Anthony, Eindhoven (NL); CHEUNG, Kit Man Lisa, Eindhoven (NL)
(74) Representative: Philips Intellectual Property & Standards

(57) **Abstract**

The invention provides a flexible cap device for placement on a subject's head and delivering light therapy to the subject's scalp, with a flexible support spine, and flexible elongate strips off-shooting from this support spine which can be bent by the subject to grip onto their scalp, in much the same manner as a grasping hand with fingers. By employing a plurality of flexible strips that are configured to distribute treatment-light received by a light input interface from one or more treatment-light sources, that may or may not be included in the flexible cap device, the unique structure of this device can be utilized to provide treatment-light therapy to a wide variety of scalp shapes and sizes.

## Description

### FIELD OF THE INVENTION

This invention relates to flexible cap devices for placement on a subject's scalp. More specifically, this invention relates to the field of flexible cap devices for delivering light-therapy to a subject's scalp.

### BACKGROUND OF THE INVENTION

Approximately 40% of adults suffer from persistent flakes on the scalp. These flakes can originate due to a variety of causes including but not limited to: dandruff; dry scalp skin; and inflammatory skin diseases such as seborrheic dermatitis and psoriasis. Flakes are generally visible on the scalp and the hair, and often shed onto the shoulders, which pose an issue for many of those affected. In all cases of flakes on the scalp, the epidermis of the skin is perturbed, with a high turnover rate and incomplete differentiation of keratinocytes, which causes the resulting corneocytes to shed not as separate cells but as clumps, known colloquially as flakes.

In the case of dandruff, the underlying cause is an oily scalp in combination with the overpopulation of a commensal fungus known as Malassezia which feeds on sebum and produced free fatty acids that irritate the scalp and induce an immune response leading to the aforementioned perturbation of the epidermis. In the case of flakes from a dry scalp, the low level of sebum cannot prevent loss of moisture from the epidermis, resulting in a compromised barrier function and immune response resulting in a similar perturbation of the epidermis. Finally, in the case of psoriasis, seborrheic dermatitis and various other skin diseases, the underlying inflammation is the cause of perturbation of skin physiology associated with scales and flakes.

Treatment and prevention of recurring flakes is a high priority issue in the field of beauty products. The application of blue-light to the scalp is emerging as a promising candidate for prevention, though there are still significant issues to address regarding how best to apply blue-light to the scalp.

Hair loss is also a major concern for a substantial portion of the population, primarily men but also women. It has been found that the application of red-light to the scalp can help prevent hair loss and also stimulate hair regrowth.

A major issue, however, is that heads come in all shapes and sizes, and any device for treatment of the scalp via light-therapy, be that blue-light or red-light would need to fit all, whilst still being relatively comfortable to wear and without falling off the subject's head. The blue or red light also needs to be distributed across a substantial portion of the scalp to be effective.

### SUMMARY OF THE INVENTION

The invention is defined by the claims.

According to examples in accordance with an aspect of the invention, there is provided a flexible cap device for placement on a subject's head and delivering light therapy to the subject's scalp, comprising:
a flexible support spine; and
a plurality of elongate flexible strips connected to the spine, wherein the elongate axis of each strip extends away from the spine to form a non-zero angle with the spine;
a light input interface configured to receive treatment-light from one or more treatment-light sources; and
wherein the strips are configured, in use, to distribute the received treatment-light to the subject's scalp.

The invention proposes the use of a flexible shape (resembling a fish-bone structure) with a flexible support spine, and flexible elongate strips off-shooting (i.e. extending or projecting) from this spine which can be bent by the subject to grip onto their scalp, in much the same manner as a grasping hand with fingers. This allows the cap device to omit a strap, which subjects may dislike and/or find uncomfortable, whilst still retaining a suitable grip on the subject's head. Also, embodiments may be flexible enough to fit any shape or size head.

By employing a plurality of flexible strips that are configured to distribute to the subject's scalp treatment-light received via a light input interface from one or more treatment-light sources (which may or may not be included in the flexible cap device), embodiments may be utilized to provide light therapy to the scalp of a subject, regardless of the shape and size of their head. The strips may distribute light to the subject's scalp in various ways. For example, the elongate strips may be made of a transparent or translucent material and may be fed treatment-light via an optical fiber in light-communication with the light input interface. The light input interface may, for example, comprise one or more apertures in each strip within which one or more treatment-light sources can be positioned to shine directly on the subject's scalp. In some embodiments, the strips may include projections (e,g. on their underside) that may comprise wave-guides or light-scatterers, the projections being in light-communication with the light input interface. Treatment-light may also be referred to as colored-light. Treatment-light is not merely any light containing suitable treatment wavelengths, but rather light that only contains light within one or more specific wavelength bands for treatment of specific issues. For instance, treatment-light may consist of blue-light or red-light or a combination of both.

In other words, by combining both a flexible yet gripping frame with suitable configurations for distributing treatment-light to the scalp of a subject, a cap device is proposed that may provide light therapy to a subject regardless of their head size and shape. The same cap device may thus be suitable for children and adults of all sizes, and even those with deformities such as lumps on the scalp. This treatment-light may be blue-light for the treatment of flakes or red-light for the treatment of hair loss, for example.

Embodiments may be based on the realization that a flexible support spine with flexible elongate strips, the elongate axis of each strip extending away from the spine to form a non-zero angle with the spine, provides a structure capable of gripping onto the subject's scalp, and may be adjustable to fit whatever size and shape scalp the subject has. The elongate axis may be defined as the axis along which the strip is elongated, in other words, the axis in parallel to the strip's longest side. The non-zero angle may be an angle within a range of 1-90 degrees.

The strips may extend away from the spine on both sides of the spine to form a fish-bone structure, or they may extend away from the spine on only one side of the spine to form a headband-type structure.

The light input interface may comprise one or more apertures configured to couple with one or more treatment-light sources. This may allow treatment-light sources to be directly integrated into the cap device and also allow for the exchanging of treatment-light sources, in order to, for example, switch blue-light sources to red-light sources, or to replace a broken treatment-light source.

The cap device may further comprise one or more treatment-light sources positioned in the one or more apertures of the light input interface. This allows the cap device to include the treatment-light sources from which the light input interface is configured to receive treatment-light, thereby providing an all-in-one solution suitable for scalp light-therapy.

The light input interface may comprise one or more apertures in each strip, and the cap device may further comprise one or more treatment-light sources positioned in each of the one or more apertures in each strip. This may allow treatment-light sources to be directly integrated into each strip.

The received treatment-light may be either blue-light, having a wavelength within a wavelength range of 400-470nm, or red-light, having a wavelength within a range of 600-700nm, or a combination of red and blue light within these ranges. These wavelength bands have been found to be the most effective for their respective therapies. The received treatment-light may also comprise both blue-light and red-light, having one or more wavelengths within each range of 400-470nm and 600-700nm for treating both flakes and hair loss at the same time.

A strip may comprise one or more projections that that are configured to contact the subject's scalp in use. These projections may then be utilized to aid in the distribution of light to the subject's scalp, or for an alternative purpose such as the massaging of the subject's scalp. This would allow the cap device, in use, to treat both flakes and promote hair regrowth at the same time.

The projections may be in light-communication with the light input interface and may be configured to distribute the treatment-light to the subject's scalp, and preferably the projections may comprise waveguides for guiding the received treatment-light to the surface of the subject's scalp, or light-scatterers for scattering the received treatment-light towards the surface of the subject's scalp. This may allow a more precise distribution of treatment-light to the subject's scalp so that it can be ensured that at least a substantial portion of the subject's scalp is receiving the light therapy. A suitable waveguide structure may, for example, be groups of hollow bristles or optical fibers, in which case there may be hundreds of projections per strip to ensure a wide distribution of the treatment-light. A suitable light-scattering structure may, for example, be a diffraction grating or a scattering filter in order to ensure a wide distribution of the treatment-light.

Each of the plurality of strips may be bendable independently from one another. This may allow for total flexibility of the structure and enhance the grip and fit on any head shape or size.

The cap device may be configured so that, in use, the spine extends substantially parallel to the coronal axis of the subject's scalp, and the plurality of flexible strips extend substantially parallel to the sagittal axis of the subject's scalp. This has been found to be a useful orientation for gripping the subject's scalp.

The cap device may be configured so that, in use, the spine extends substantially parallel to the sagittal axis of the subject's scalp, and the plurality of flexible strips extend substantially parallel to the coronal axis of the subject's scalp. This has been found to be another useful orientation for gripping the subject's scalp.

Due to the cap device's flexible nature, it may be capable of gripping onto the scalp and providing light therapy in either orientation.

The cap device may be configured so that, in use, the spine extends substantially across the subject's forehead or substantially across the subject's crown, and the plurality of strips may be configured to extend substantially across the subject's scalp. This may result in headband-like structure with the strips extending out only from one side of the support spine.

The plurality of strips may comprise a first and second group of strips, and the first and second groups of strips may be configured so that, in use, the first group of strips extend towards the anterior portion of the subject's scalp, and the second group of strips extend towards the posterior portion of the subject's scalp. The extension of strips towards the front and rear of the subject's scalp, regardless of the spine orientation, has been found to increase the grip of the cap device on a subject's scalp whilst also allowing for distribution of the treatment-light at the front and rear of the subject's scalp.

The cap device may further comprise two sideburn strips configured so that, in use, each sideburn strip substantially covers one of the subject's sideburns respectively. For example, when the support spine is orientated substantially parallel to the coronal axis of the patient's scalp, one of the outermost strips from each side of the flexible cap may comprise an elongated sideburn strip extending substantially perpendicular to the plurality of strips and substantially parallel to the spine, configured so that, when the cap device is placed on the patient's scalp, each sideburn strip substantially covers one of the patient's sideburns.

The spine may be bendable in such a way that it may be bent by the subject to follow the contour of the subject's scalp. This may allow for an enhanced grip on the subject's scalp regardless of the size or shape of their head.

There is also provided an item of headwear comprising any herein disclosed cap device.

Thus, there may be proposed concepts for a flexible cap device for placement on a subject's head and delivering treatment-light therapy to a subject's scalp.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an underside view of a flexible cap device according to an embodiment;
Fig. 2 shows a sideview of a flexible cap device according to an embodiment, wherein the flexible cap is positioned on the head of a user;
Fig. 3 shows a front view of a flexible cap device according to another embodiment, wherein the flexible cap is positioned on the head of a user;
Fig. 4 shows a sideview of a flexible cap device according to another embodiment, wherein the flexible cap is positioned on the head of a user;
Fig. 5 shows a plan view of a flexible cap device according to another embodiment; and
Fig. 6 shows an underside view of a flexible cap device according to another embodiment.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a flexible shape with a flexible support spine and flexible elongate strips off-shooting (i.e. extending or projecting) from this support spine which can be bent by the subject to grip onto their scalp, in much the same manner as a grasping hand with fingers. By employing a plurality of flexible strips that are configured to distribute treatment-light received by a light input interface from one or more treatment-light sources, that may or may not be included in the flexible cap device, the unique structure of this device can be utilized to provide treatment-light therapy to a wide variety of scalp shapes and sizes. The subject may also be referred to as a user or a patient, or any other suitable term.

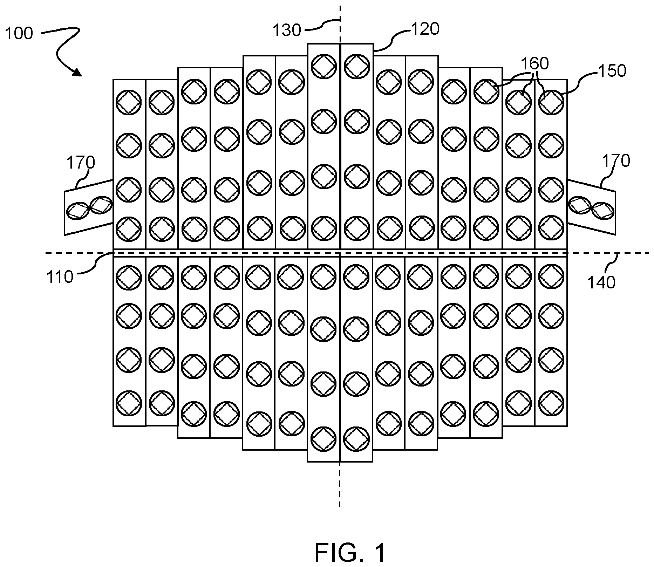

Fig. 1 shows an underside view of a flexible cap device 100 according to a proposed embodiment. That is, the illustration of Fig. 1 depicts the side of the flexible cap that faces the subject's scalp when in use and placed upon the subject's scalp. The flexible cap device 100 comprises a flexible support spine 110, here orientated substantially parallel to the coronal axis 140 of the subject's scalp, and a plurality of elongate flexible strips 120 connected to the spine. The strips are all at right angles with the spine (i.e. perpendicular to the spine) and thus configured to be substantially parallel with the sagittal axis 130 of the subject's scalp when in use.

The elongate axis of each strip 120 is linear and forms a ninety-degree angle with the support spine 110. However, this is only exemplary and is not required for the invention to function. The elongate axis may be linear or curved and may form any angle with the spine between 1-90 degrees.

The plurality of strips 120 comprise first and second groups of strips. The first group comprises strips above the spine 110 in Fig. 1 and so extend towards the anterior portion of the subject's scalp. The second group comprise strips below the spine 110 in Fig. 1 and so extend towards the posterior portion of the subject's scalp. This ensures a good grip on the subject's scalp and also a wide distribution of the treatment-light across a substantial portion of the front and back of the subject's scalp. The substantial portion may be more than 10% of the subject's scalp. Preferably, it may be more than 20%, 30%, 40% or 50% of the subject's scalp. Even more preferably, it may be more than 60%, 70%, 80% or 90% of the subject's scalp.

The strips 120 are configured to distribute received treatment-light to the subject's scalp. In this embodiment, this is achieved via each strip comprising a plurality of apertures, which together form the light input interface 160, a treatment-light source (not shown) being positioned within each aperture. These treatment-light sources may, for instance, comprise LEDs configured to produce either blue-light or red-light. For LEDs configured to produce blue-light, the light may have a wavelength within a wavelength range of 400-470nm - these wavelengths having been found to be the most effective for blue-light therapy intended to treat flakes. For LEDs configured to produce red-light, the light may have a wavelength within a wavelength range of 600-700nm, or preferably between 630-660nm - these wavelengths having been found to be the most effective for red-light therapy intended to prevent hair loss and to promote hair regrowth.

Multiple treatment-light sources may also be positioned within each aperture. These multiple treatment-light sources may be configured to produce light of the same wavelength as one other, or may be configured to produce light of different wavelengths, e.g. one blue-light source and one red-light source per aperture.

Each strip 120 also has a plurality of projections 150 that are configured to contact the subject's scalp in use. The projections are each in light-communication with the light input interface 160.

In this embodiment, light-communication between the projections 150 and the light input interface 160 is achieved via each projection being substantially below their own respective aperture. In use, the projections are thus between the light input interface, i.e. the apertures, and the subject's scalp. Light-communication may also be referred to as optical-communication, and is merely intended to convey that the cap device is so configured as to allow light to travel between the light input interface and a projection on a strip. This may be achieved in a variety of ways, for example, the use of optical fibers, waveguides, an arrangement of reflective elements, or direct contact.

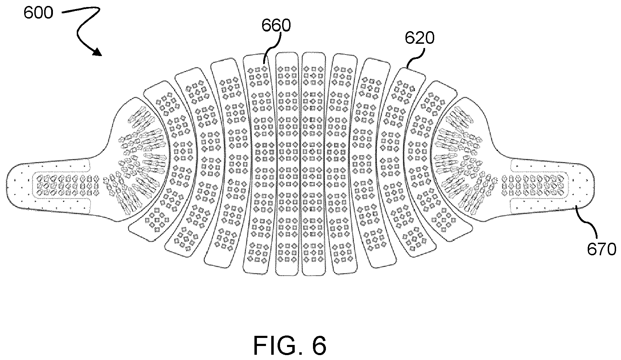

The projections 150 are configured to distribute the received treatment-light to the subject's scalp. This may be achieved by the projections comprising waveguides for guiding the treatment-light from the treatment-light sources to the subject's scalp. For example, a projection may comprise a group of hollow bristles that guide treatment-light directly from the treatment-light sources, positioned in each aperture of the light input interface 160 down to the subject's scalp through the hair. An example of this can be seen in Fig. 6. The projections may alternatively comprise light-scatterers for scattering the light from the treatment-light sources towards the subject's scalp. This may be achieved, for example, by making the projections light-scattering structures.

The cap device 100 also includes two sideburn strips 170 configured so that, in use, i.e. when the cap device is placed upon the subject's scalp, each sideburn strip substantially covers one of the subject's sideburns respectively. This is achieved in this embodiment by having one of the outermost elongate strips 120 from each side of the cap device comprise an elongated sideburn strip extending substantially perpendicular to the plurality of flexible strips and substantially parallel to the spine, configured so that, in use, each sideburn strip substantially covers one of the subject's sideburns respectively. This facilitates the application of treatment-light therapy to the subject's sideburns.

The flexible cap 100, in some embodiments, may be so configured as to hide the support spine 110 when viewing the flexible cap from its underside. This may achieve the aim of having the strips 120 closer together and so distributing light more evenly across a subject's scalp.

The plurality of strips 120 are of different lengths so as to accommodate the general narrowing of scalps towards the anterior and posterior of the scalp. The strips are arranged such that the length of the strips descend the further from the center of the support spine 110 they are positioned. In some embodiments, the strips may also have tapered ends so that the length of strips make a smooth gradient. In other embodiments, the strips may all be of the same length.

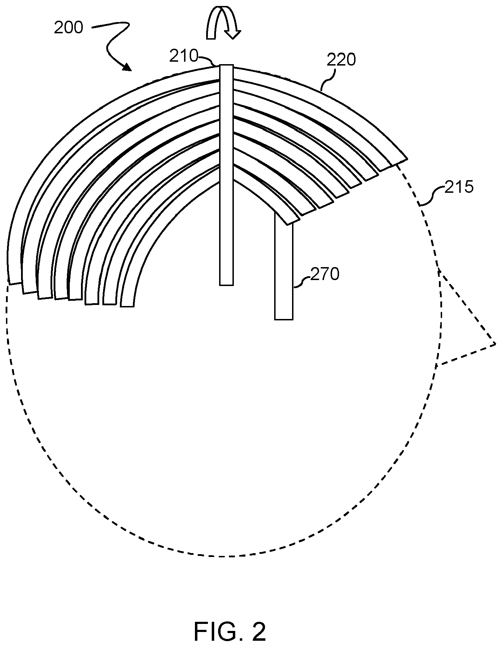

Fig. 2 shows a sideview of a flexible cap device 200 positioned on the head 215 of a subject according to an exemplary embodiment. In this embodiment, the cap device is configured so that, in use, the spine 210 extends substantially parallel to the coronal axis of the subject's scalp, and the plurality of elongate strips 220 extend substantially parallel to the sagittal axis of the subject's scalp. This has been found to be a useful orientation for gripping the subject's scalp. In this embodiment the strips are different lengths to one another. In other embodiments, however, the strips may all be of the same length, different lengths, or split up into subgroups of strips of the same lengths.

In this embodiment, the plurality of strips 220 comprise first and second groups, and the first and second groups of strips are configured so that the first group of strips extend towards the anterior portion of the subject's scalp, i.e. the strips extending to the right of the Fig, and the second group of strips extend towards the posterior portion of the subject's scalp, i.e. the strips extending to the left of the Fig. This structure has been found to increase the grip of the cap device 200 on a subject's scalp whilst also allowing for distribution of the treatment-light at the front and rear of the subject's scalp.

The support spine 210 is bendable and has been bent to follow the contour of the subject's scalp. This allows for enhanced grip on the subject's scalp regardless of the size and shape of their head 215.

Each of the plurality of strips 220 is bendable independently from one another, and as can be seen in the Fig, the strips have each been bent to follow the contour of the subject's head 215. This allows for total flexibility of the structure and enhanced the grip and fit on any head shape or size.

The cap device 200 also comprises two sideburn strips 270 (only one of which is visible) configured so that, in use, each side burn strip substantially covers one of the subject's sideburns respectively. This facilitates the application of treatment-light therapy to the subject's sideburns.

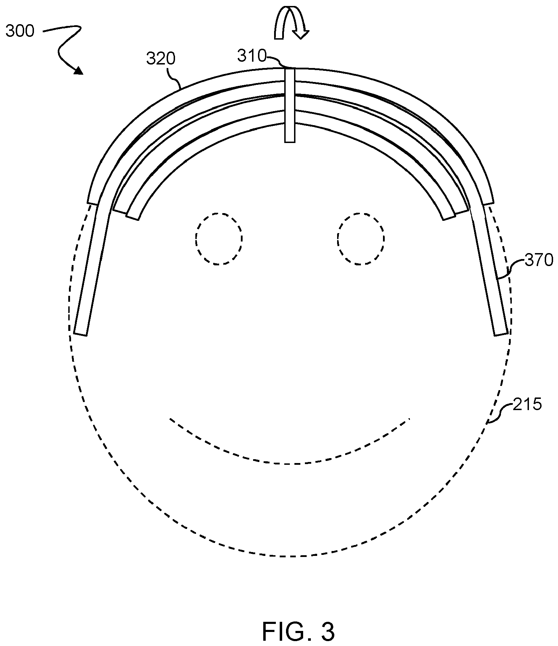

Fig. 3 shows a front-view of a flexible cap device 300 on the head 215 of a subject according to another exemplary embodiment. In this embodiment, the support spine 310 is orientated substantially parallel to the sagittal axis of the subject's scalp, and the plurality of strips are orientated substantially parallel to the coronal axis of the subject's scalp, extending either side of the support spine.

The sideburn strips 370 are configured as to solely comprise longer elongate strips 320, as compared to the other elongate strips, positioned in such a way so that when the cap device 300 is placed upon the subject's scalp, i.e. when in use, they each substantially cover one of the subject's sideburns respectively.

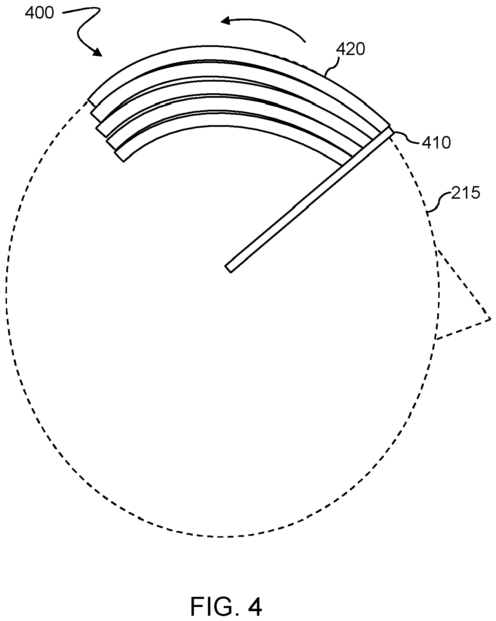

Fig. 4 shows a sideview of a flexible cap device 400 on the head 215 of a subject according to another exemplary embodiment. In this embodiment, the support spine 410 extends substantially across the subject's forehead, and the plurality of strips 420 are configured to extend substantially across the subject's scalp, extending substantially perpendicular to the spine and towards the posterior portion of the subject's scalp. The strips may extend further than is shown to cover a more substantial portion of the subject's scalp. In some embodiments, the strips may also be secured at their ends by a separate support clip in order to enhance the flexible cap's grip on the subject's head.

In another embodiment, the support spine may extend substantially across the subject's crown, i.e. across the back of the subject's head, and the elongate strips may then extend towards the anterior portion of the subject's scalp, i.e. towards the front of the subject's head.

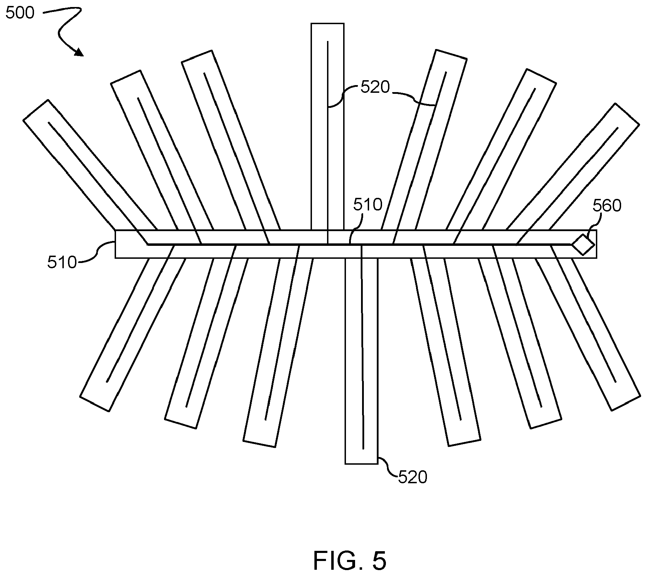

Fig. 5 shows an above view of a flexible cap device 500 according to an alternate embodiment. In this embodiment, the elongate axis of each strip 520 extends away from the spine and forming a different non-zero angle with the spine 510, so as to form a fan-like structure.

The light input interface 560 comprises a single light housing on the spine 510 configured to receive treatment-light from one or more treatment-light sources. This may be achieved by housing one or more treatment-light sources within the light housing, or alternatively, by being in light-communication with one or more treatment-light sources separate to the cap device 500. A main optical fiber 510 in light-communication with the light input interface may then extend along the support spine, from which subsidiary optical fibers 520 may then extend along the strips 520. In this embodiment, the strips may comprise transparent or translucent materials thereby allowing treatment-light from the subsidiary optical fibers to be distributed to the subject's scalp through the strips. Alternatively, the subsidiary optical fibers may go directly through apertures in the strips to reach the subject's scalp. The options presented as to how to distribute the received treatment-light to the subject's scalp are merely examples, and any suitable configuration would be sufficient.

In other embodiments, the light input interface may be an aperture, in which a treatment-light source may be positioned. The main optical fiber may then run through the support spine, and the subsidiary optical fibers may then run through the strips.

In other embodiments, the light input interface may comprise a plurality of apertures or light housings along the support spine. Each of the plurality of apertures or light housings may then be in light-communication with one or more one or more strips for subsequent distribution of the received treatment-light to the subject's scalp.

Fig. 6 shows an underside view of a flexible cap device 600 according to another embodiment. In this embodiment, the support spine is hidden from the underside view but runs through the center of the device, configured so that, in use, the spine would run substantially parallel to the coronal axis of the subject's scalp. The elongate strips 620 are project from the support spine and are substantially the same length. Sideburn strips 670 are connected to the outermost strips on each side of the cap device, and configured so that, in use, they each substantially cover one of the subject's sideburns.

The light input interface 660 comprises an array of apertures (not visible) in each strip. A treatment-light source (not visible) is positioned within each aperture. Positioned below each aperture, i.e. closer to the subject's scalp when in use, are projections 660. In this embodiment, the projections 660 comprise groups (or tufts) of bristles, each group being positioned directly below each aperture and thus each treatment-light source. The bristles are configured as waveguides. The bristles may be hollow. The bristles are configured to contact the subject's scalp when the cap device 600 is in use, and guide the received treatment-light directly to the subject's scalp, ensuring a distribution of treatment-light across a substantial portion of the subject's scalp.

This embodiment, along with any other disclosed herein, may form part of an item of headwear. This item of headwear may further comprise foam to enhance the subject's comfort in use.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A flexible cap device (100) for placement on a subject's head and delivering light therapy to the subject's scalp, comprising:
a flexible support spine (110);
a plurality of elongate flexible strips (120) connected to the spine, wherein the elongate axis of each strip extends away from the spine to form a non-zero angle with the spine; and
a light input interface (160) configured to receive treatment-light from one or more treatment-light sources,
wherein the strips are configured, in use, to distribute the received treatment-light to the subject's scalp.

2. The cap device of claim 1, wherein the light input interface (160) comprises one or more apertures configured to couple with one or more treatment-light sources.

3. The cap device of claim 2, wherein the cap device further comprises one or more treatment-light sources positioned in the one or more apertures of the light input interface (160).

4. The cap device of claim 1, wherein the light input interface (160) comprises one or more apertures in each strip (120), and wherein the cap device (100) further comprises one or more treatment-light sources positioned in each of the one or more apertures in each strip.

5. The cap device of any of claims 1 to 4, wherein the received treatment-light is either blue-light, having a wavelength within a wavelength range of 400-470nm, or red-light, having a wavelength within a range of 600-700nm, or a combination of red and blue light within these ranges.

6. The cap device of any of claims 1 to 5, wherein a strip (120) comprises one or more projections (150) that are configured to contact the subject's scalp in use.

7. The cap device of claim 6, wherein the projections (150) are in light-communication with the light input interface (160) and are configured to distribute the treatment-light to the subject's scalp, and preferably wherein the projections (150) comprise wave-guides for guiding the received treatment-light to the surface of the subject's scalp, or light-scatterers for scattering the received treatment-light towards the surface of the subject's scalp.

8. The cap device of any of claims 1 to 7, wherein each of the plurality of strips (120) is bendable independently from one another.

9. The cap device of any of claims 1 to 8, wherein the cap device is configured so that, in use, the spine (110) extends substantially parallel to the coronal axis (140) of the subject's scalp, and the plurality of flexible strips (120) extend substantially parallel to the sagittal axis (130) of the subject's scalp.

10. The cap device of any of claims 1 to 8, wherein the cap device is configured so that, in use, the spine (110) extends substantially parallel to the sagittal axis (130) of the subject's scalp, and the plurality of flexible strips (120) extend substantially parallel to the coronal axis (140) of the subject's scalp.

11. The cap device of any of claims 1 to 8, wherein the cap device is configured so that, in use, the spine (110) extends substantially across the subject's forehead or substantially across the subject's crown, and the plurality of strips (120) are configured to extend substantially across the subject's scalp.

12. The cap device of any of claims 1 to 11, wherein the plurality of strips (120) comprises a first and second group of strips, and wherein the first and second groups of strips are configured so that, in use, the first group of strips extend towards the anterior portion of the subject's scalp, and the second group of strips extend towards the posterior portion of the subject's scalp.

13. The cap device of any of claims 1 to 12, wherein the cap device further comprises two sideburn strips (170) configured so that, in use, each side burn strip substantially covers one of the subject's sideburns respectively.

14. The cap device of any of claims 1 to 13, wherein the spine (110) is bendable in such a way that it may be bent by the subject to follow the contour of the subject's scalp.

15. An item of headwear comprising the cap device of any of claims 1 to 14.
